# EUROPEAN PATENT APPLICATION

(11) **EP 3 839 033 A1**
(43) Date of publication of application: **23.06.2021**
(21) Application number: 19218710.2
(22) Date of filing: 20.12.2019
(51) Int. Cl.: C12M 1/12, C12M 1/32

(54) **DEVICE FOR THE THREE-DIMENSIONAL CULTURE OF CELLS**

(71) Applicant: Comenius University in Bratislava, 814 99 Bratislava 1 (SK)
(72) Inventor: Strnadel, Jan, 027 44 Tvrdosin (SK)
(74) Representative: Schiweck Weinzierl Koch Patentanwälte Partnerschaft mbB

(57) **Abstract**

The present invention relates to devices for three dimensional culture of cells in a matrix (M1) which comprise at least one mesh inserted in at least one plate and at least one cover (C1) around the at least one mesh; which can be fluid non-permeable. Furthermore, the present invention relates to methods for three dimensional culture of cells in a matrix (M1), which comprise installing at least one of the at least one mesh in at least one of the at least one plate.

## Description

### FIELD OF THE INVENTION

The present invention relates to devices and methods for three dimensional culture of cells.

The present invention relates to devices for three dimensional culture of cells in a matrix (M1) which comprise at least one mesh inserted in at least one plate and at least one cover (C1) around the at least one mesh; which may be fluid non-permeable.

Furthermore, the present invention relates to methods for three dimensional culture of cells in a matrix (M1), which comprise installing at least one mesh in at least one plate.

### BACKGROUND OF THE INVENTION

Three dimensional (3D) culture of primary cancer cells or cell lines embedded in a matrix for 3D culture of the cells is a technique commonly used in research labs.

In the known methods, fresh cell culture medium is added frequently or whole medium of the cultured cells is changed. However, complete change of medium is tricky, in particular for media containing gelatinous extract of extracellular matrix proteins isolated from Engelbreth-Holm-Swarm (EHS) mouse sarcomas, because of semi-solid nature of gelatinous extract of extracellular matrix proteins isolated from Engelbreth-Holm-Swarm (EHS) mouse sarcomas. Often, small pieces of gelatinous extract of extracellular matrix proteins isolated from Engelbreth-Holm-Swarm (EHS) mouse sarcomas are sucked into the pipette and cells are lost during the medium change.

According to Fig. 1 in US 5,578,490 A, a cell culture plate with a system for lateral diffusion of molecules across a barrier membrane is disclosed, comprising a base consisting of two or more flat-bottomed open-topped wells, denominated the reservoir wells, each containing one or more wells of smaller size (diffusion wells), likewise open-topped and sharing the same flat base, characterized in that each of the diffusion wells communicates with the larger reservoir well which houses it via one or several slits located in the lateral wall(s) of the diffusion well(s), and in that each slit is covered by a semipermeable membrane that permits the passage of soluble substances while retaining insoluble particles and cells; and a removable lid. The cells deposited in the diffusion well(s) may interchange their metabolic products with the corresponding reservoir well containing cell culture medium or other cells. The problem caused by other devices wherein the diffusion of substances occurs between a higher compartment (which usually contains the cells) and a lower compartment, separated by a barrier membrane, making difficult the microscopic observation of the cells and the manipulation thereof, is thus solved. The fields of application include various branches of biology, such as immunology, pharmacology, toxicology, microbiology and parasitology. The disclosed diffusion system comprises two flat-bottomed open-topped wells, which communicate via two slits, which leads to relative slow exchange of nutrients and gases between the media and two wells.

A cell culture well insert is disclosed in WO 2016/069917 A1, which may include a frame defining a first open end, a second open end, and at least one support extending there between. The cell culture well insert may also include a fluid permeable mesh coupled to the frame and disposed across the second open end. The mesh may define pores that have an average pore size in a range from 10 micrometers to 100 micrometers. The cultured cells/spheroids assemble at the bottom of the well below the mesh. Such a design results in disturbance of infrared, visible, ultraviolet or fluorescent light source by the mesh material in the path of the light, while users take images of cells/spheroids. Furthermore, this designed insert restrains contact of the cell culture medium and cell/spheroids entrapped inside the insert, due to production of gases during the cell culture process.

Thus, there is a need for further 3D cell culture methods and devices.

### SUMMARY OF THE INVENTION

The invention is directed to a device for three dimensional (3D) culture of cells in a matrix (M1), which comprises:
(a) at least one mesh inserted in at least one plate
(b) at least one cover (C1) around the at least one mesh; which preferably is fluid non-permeable.

Further, the invention is directed to a method for three dimensional (3D) culture of cells in a matrix (M1) comprising the steps:
(a) installing at least one mesh in at least one plate;
(b) inserting at least one cover (C1) around the at least one mesh (20);
(c) adding at least one matrix (M1) inside at least one of the at least one mesh (20) and/or at least one of the at least one mesh (20) may comprise at least one matrix (M1);
(d) removing at least one of the at least one cover (C1) after solidifying of the at least one matrix (M1);
(e) adding at least one matrix (M2) into at least one of the at least one plate (40) outside the area enclosed by at least one of the at least one mesh (20).

Compared with the prior art, the present invention has the following beneficial effects:

The mesh in which matrices M1 and M2 are in contact through from the lateral direction of the mesh allows faster exchange of nutrients and gases between matrices M1 and M2.

The mesh disclosed herein is "imprinted on the plate" and it is not placed like the conventional devices, on bottom of a well. Thus, matrices M1 and M2 are in contact through the mesh in lateral direction. Therefore, it is enabled to take images of cells/spheroids without disturbance of infrared, visible, ultraviolet or fluorescent light by a mesh material, placed in the path of the light source.

The disclosed method and device allows minimizing number of manipulation steps and number of devices, which are in contact with the cell culturing media. Therefore, the risk of infection and contamination of the cell culture media, which is closely linked with manipulation and placing of external devices, is eliminated by using the device as well as the method disclosed herein.

The device as well as method disclosed herein minimizes loss of valuable cultured cells, which occurs while changing of the culture media via conventional methods like for example using a pipette.

### BRIEF DESCRIPTION OF THE DRAWINGS

Figure 1 is a schematic diagram of the 3D MESH according to one of embodiment of the invention.
Figure 2 is a schematic diagram of the method of 3D cell culture according to one of embodiment the invention.

### DETAILED DESCRIPTION OF THE INVENTION

The solution of the present invention is described in the following, illustrated in the Figures and reflected in the claims.

It must be noted that as used herein, the singular forms "a", "an", and "the", include plural references unless the context clearly indicates otherwise. Thus, for example, reference to "a plate" includes one or more of such different plates and reference to "the method" includes reference to equivalent steps and methods known to those of ordinary skill in the art that could be modified or substituted for the methods described herein.

Unless otherwise indicated, the term "at least" preceding a series of elements is to be understood to refer also to every element in the series. Those skilled in the art will recognize, or be able to ascertain using no more than routine experimentation, many equivalents to the specific embodiments of the invention described herein. Such equivalents are intended to be encompassed by the present invention.
- When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

In the context of this invention, the term "providing" means making available in any practical useful way for usage. This includes the actual preparation by combination of the ingredients and solvents, as well as the use of a potentially commercially or otherwise readily available solution.

The present invention relates to devices and methods for three dimensional culture of cells.

The invention is directed to a device for three dimensional (3D) culture of cells in a matrix (M1), comprising:
(a) at least one mesh (20) inserted in at least one plate (40)
(b) at least one cover (C1) around the at least one mesh (20); which preferably is fluid non-permeable.

The matrix (M1) may be typically represented by defined synthetic matrix for 3D culture, provided by biotech companies and cells to be cultured. The defined synthetic matrix, which is commercially available, can be replaced by a mixture of cell culture medium (as discussed above) and gelatinous extract of extracellular matrix proteins isolated from Engelbreth-Holm-Swarm (EHS) mouse sarcomas.

The mixture of cells, cell culture medium and gelatinous extract of extracellular matrix proteins isolated from Engelbreth-Holm-Swarm (EHS) mouse sarcomas solidifies at around 10 °C. Thus, during preparation the mixture of cells, cell culture medium and gelatinous extract of extracellular matrix proteins isolated from Engelbreth-Holm-Swarm (EHS) mouse sarcomas may be chilled (preferably at temperature 1 to 8 °C, more preferably at 3 to 5 °C, most preferably at 4 °C, or by keeping the mixture on the ice) in order to be able to mix cells with cell culture medium and the gelatinous extract of extracellular matrix proteins isolated from Engelbreth-Holm-Swarm (EHS) mouse sarcomas. Once mixture is ready, the solidification of the mixture may be induced by placing the mixture in incubator at temperature preferably 25 to 45 °C, more preferably at 30 to 40 °C, most preferably at 35 to 39 °C or most preferably at 37 °C.

Preferably, the final concentration of gelatinous extract of extracellular matrix proteins isolated from Engelbreth-Holm-Swarm (EHS) mouse sarcomas in matrix (M1) is 2 to 5 mg/ml. More preferably, 2 to 3mg/ml.

Preferably, the final concentration of gelatinous extract of extracellular matrix proteins isolated from Engelbreth-Holm-Swarm (EHS) mouse sarcomas in matrix (M1) is 2, 2.5, 2.6, 2.7, 2.8, 2.9, 3, 3.1, 3.2, 3.3, 3.4, 3.5, 4, 4.5 or 5 mg/ml, more preferably 3 mg/ml.

The cells to be cultured may be Pluripotent stem cells (induced pluripotent stem cells), adult stem cells (mesenchymal stem cells, dental pulp stem cells), cancer cells and cancer stem cells (primary cancer cells or commercially available cancer cells isolated from fresh tumor samples).

The concentration may be assay dependent. Preferably the concentration range of the cells to be cultured is from one thousand cells per ml to one hundred million cells per ml, more preferably, the concentration range of cells to be cultured is ten thousand to ten million cells per ml, most preferably the concentration range of cells to be cultured is one hundred thousand to one million cells per ml.

Preferably, the concentration of cells to be cultured is 1000, 10000, 100000, 200000, 300000, 400000, 500000, 600000, 700000, 800000, 900000, 1000000 or 10000000 cells per ml.

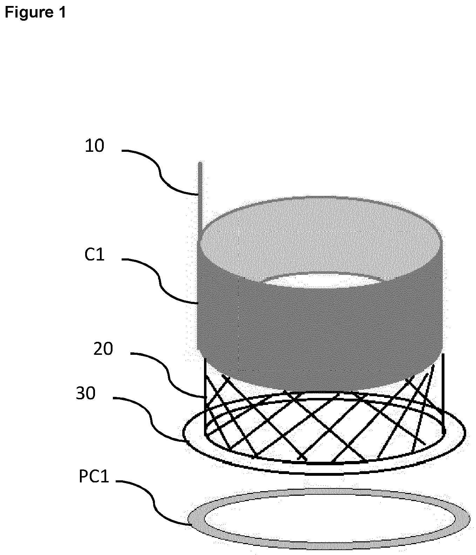

Fig. 1 shows one embodiment of the inventive device.

It should be understood that several matrices (M1) may be added in at least one/or at each one of mesh (20).

At temperatures between 20 °C to 43 °C, the matrix (M1) may be solidified.

At temperatures between 25 °C to 42 °C, the matrix (M1) may be solidified.

At temperatures between 30 °C to 41 °C, the matrix (M1) may be solidified.

At temperatures between 31 °C to 40 °C, the matrix (M1) may be solidified.

At temperatures between 32 °C to 39 °C, the matrix (M1) may be solidified.

At temperatures between 33 °C to 38 °C, the matrix (M1) may be solidified.

At temperatures between 34 °C to 37 °C, the matrix (M1) may be solidified.

At temperatures between 36 °C to 38 °C, the matrix (M1) may be solidified.

At temperatures between 36 °C to 39 °C, the matrix (M1) may be solidified.

At temperatures between 37 °C to 38 °C, the matrix (M1) may be solidified.

At temperatures between 36 °C to 37 °C, the matrix (M1) may be solidified.

At 37 °C, the matrix (M1) may be solidified.

Wherein the term "solidified" means that the matrix (M1) would keep its form even if the mesh and/or the cover (C1) is removed.

At any of the temperature ranges of 20 °C to 43 °C, 25 °C to 42 °C, 30 °C to 41 °C, 31 °C to 40 °C, 32 °C to 39 °C, 33 °C to 38 °C, 34 °C to 37 °C, 36 °C to 38 °C, 36 °C to 39 °C, 37 °C to 38 °C , 36 °C to 37 °C or at 37 °C, the matrix (M1) may be solidified after 0 to 120 minutes.

At any of the temperature ranges of 20 °C to 43 °C, 25 °C to 42 °C, 30 °C to 41 °C, 31 °C to 40 °C, 32 °C to 39 °C, 33 °C to 38 °C, 34 °C to 37 °C, 36 °C to 38 °C, 36 °C to 39 °C, 37 °C to 38 °C , 36 °C to 37 °C or at 37 °C, the matrix (M1) may be solidified after 10 to 110 minutes.

At any of the temperature ranges of 20 °C to 43 °C, 25 °C to 42 °C, 30 °C to 41 °C, 31 °C to 40 °C, 32 °C to 39 °C, 33 °C to 38 °C, 34 °C to 37 °C, 36 °C to 38 °C, 36 °C to 39 °C, 37 °C to 38 °C , 36 °C to 37 °C or at 37 °C, the matrix (M1) may be solidified after 20 to 100 minutes.

At any of the temperature ranges of 20 °C to 43 °C, 25 °C to 42 °C, 30 °C to 41 °C, 31 °C to 40 °C, 32 °C to 39 °C, 33 °C to 38 °C, 34 °C to 37 °C, 36 °C to 38 °C, 36 °C to 39 °C, 37 °C to 38 °C , 36 °C to 37 °C or at 37 °C, the matrix (M1) may be solidified after 30 to 90 minutes.

At any of the temperature ranges of 20 °C to 43 °C, 25 °C to 42 °C, 30 °C to 41 °C, 31 °C to 40 °C, 32 °C to 39 °C, 33 °C to 38 °C, 34 °C to 37 °C, 36 °C to 38 °C, 36 °C to 39 °C, 37 °C to 38 °C , 36 °C to 37 °C or at 37 °C, the matrix (M1) may be solidified after 40 to 80 minutes.

At any of the temperature ranges of 20 °C to 43 °C, 25 °C to 42 °C, 30 °C to 41 °C, 31 °C to 40 °C, 32 °C to 39 °C, 33 °C to 38 °C, 34 °C to 37 °C, 36 °C to 38 °C, 36 °C to 39 °C, 37 °C to 38 °C , 36 °C to 37 °C or at 37 °C, the matrix (M1) may be solidified after 45 to 75 minutes.

At any of the temperature ranges of 20 °C to 43 °C, 25 °C to 42 °C, 30 °C to 41 °C, 31 °C to 40 °C, 32 °C to 39 °C, 33 °C to 38 °C, 34 °C to 37 °C, 36 °C to 38 °C, 36 °C to 39 °C, 37 °C to 38 °C , 36 °C to 37 °C or at 37 °C, the matrix (M1) may be solidified after 50 to 70 minutes.

At any of the temperature ranges of 20 °C to 43 °C, 25 °C to 42 °C, 30 °C to 41 °C, 31 °C to 40 °C, 32 °C to 39 °C, 33 °C to 38 °C, 34 °C to 37 °C, 36 °C to 38 °C, 36 °C to 39 °C, 37 °C to 38 °C , 36 °C to 37 °C or at 37 °C, the matrix (M1) may be solidified after 51 to 69 minutes.

At any of the temperature ranges of 20 °C to 43 °C, 25 °C to 42 °C, 30 °C to 41 °C, 31 °C to 40 °C, 32 °C to 39 °C, 33 °C to 38 °C, 34 °C to 37 °C, 36 °C to 38 °C, 36 °C to 39 °C, 37 °C to 38 °C , 36 °C to 37 °C or at 37 °C, the matrix (M1) may be solidified after 52 to 68 minutes.

At any of the temperature ranges of 20 °C to 43 °C, 25 °C to 42 °C, 30 °C to 41 °C, 31 °C to 40 °C, 32°C to 39 °C, 33°C to 38 °C, 34°C to 37 °C, 36°C to 38 °C, 36°C to 39 °C, 37 °C to 38 °C , 36 °C to 37 °C or at 37 °C, the matrix (M1) may be solidified after 53 to 67 minutes.

At any of the temperature ranges of 20 °C to 43 °C, 25 °C to 42 °C, 30 °C to 41 °C, 31 °C to 40 °C, 32°C to 39 °C, 33°C to 38 °C, 34°C to 37 °C, 36°C to 38 °C, 36°C to 39 °C, 37 °C to 38 °C , 36 °C to 37 °C or at 37 °C, the matrix (M1) may be solidified after 54 to 66 minutes.

At any of the temperature ranges of 20 °C to 43 °C, 25 °C to 42 °C, 30 °C to 41 °C, 31 °C to 40 °C, 32°C to 39 °C, 33°C to 38 °C, 34°C to 37 °C, 36°C to 38 °C, 36°C to 39 °C, 37 °C to 38 °C , 36 °C to 37 °C or at 37 °C, the matrix (M1) may be solidified after 55 to 65 minutes.

At any of the temperature ranges of 20 °C to 43 °C, 25 °C to 42 °C, 30 °C to 41 °C, 31 °C to 40 °C, 32°C to 39 °C, 33°C to 38 °C, 34°C to 37 °C, 36°C to 38 °C, 36°C to 39 °C, 37 °C to 38 °C , 36 °C to 37 °C or at 37 °C, the matrix (M1) may be solidified after 56 to 64 minutes.

At any of the temperature ranges of 20 °C to 43 °C, 25 °C to 42 °C, 30 °C to 41 °C, 31°C to 40 °C, 32°C to 39 °C, 33°C to 38 °C, 34°C to 37 °C, 36°C to 38 °C, 36°C to 39 °C, 37 °C to 38 °C , 36 °C to 37 °C or at 37 °C, the matrix (M1) may be solidified after 57 to 63 minutes.

At any of the temperature ranges of 20 °C to 43 °C, 25 °C to 42 °C, 30 °C to 41 °C, 31 °C to 40 °C, 32 °C to 39 °C, 33 °C to 38 °C, 34 °C to 37 °C, 36 °C to 38 °C, 36 °C to 39 °C, 37 °C to 38 °C , 36 °C to 37 °C or at 37 °C, the matrix (M1) may be solidified after 58 to 62 minutes.

At any of the temperature ranges of 20 °C to 43 °C, 25 °C to 42 °C, 30 °C to 41 °C, 31 °C to 40 °C, 32 °C to 39 °C, 33 °C to 38 °C, 34 °C to 37 °C, 36 °C to 38 °C, 36 °C to 39 °C, 37 °C to 38 °C , 36 °C to 37 °C or at 37 °C, the matrix (M1) may be solidified after 59 to 61 minutes.

At any of the temperature ranges of 20 °C to 43 °C, 25 °C to 42 °C, 30 °C to 41 °C, 31 °C to 40 °C, 32°C to 39 °C, 33°C to 38 °C, 34°C to 37 °C, 36°C to 38 °C, 36°C to 39 °C, 37 °C to 38 °C , 36 °C to 37 °C or at 37 °C, the matrix (M1) may be solidified after 60 minutes.

At around 37 °C, the matrix (M1) may be solidified after 60 minutes.

The mesh (20) may comprise a cross section, which preferably may be in contact with the plate (40), and a height.

Preferably, the mesh (20) and the cross section may comprise any shape, in which each of the dimensions of the cross section is 0.1 to 2.5 cm, more preferably 0.5 to 2.0 cm.

The mesh (20) and the cross section may comprise any shape, in which each of the dimensions of the cross section may be 0.5 cm, 0.6 cm, 0.7 cm, 0.8 cm, 0.9 cm, 1 cm, 1.1 cm, 1.2 cm, 1.3 cm, 1.4 cm, 1.5 cm, 1.6 cm, 1.7 cm, 1.8 cm, 1.9 cm or 2 cm.

A typical cell culture plate, for example a Petri dish, has preferably a height of 15 to 25 mm, more preferably 17 to 23 mm, most preferably 20 mm. Preferably the height of the mesh (20) may be less than the height of the plate (40). More preferably the height of the mesh (20) may be 8 mm to 20 mm, more preferably 10 to 20 mm, most preferably 15 mm.

Preferably the mesh (20) comprises pores with every shape, more preferably, symmetric or asymmetric shape.

Preferably each of the dimensions of the mesh (20) pores is preferably 5 to 110 µm, more preferably 10 to 100 µm.

The dimensions of the mesh (20) pores may be 10 µm, 20 µm, 30 µm, 40 µm, 50 µm, 60 µm, 70 µm, 80 µm, 90 µm, 100 µm. Larger pores may be selected when semi-solid or solidified matrix is used for spheroid culture. Mesh insert can be used also for culture of cells in 3D as free floating spheroids, in liquid medium. In such case, mesh with smaller pore size (10 or 20 µm) may be selected.

Preferably, the plate (40) may be made of clear polystyrene; or any other standard material for producing culture plates.

Preferably, the plate (40) may have a cross section and may have a height.

Preferably, each of the dimensions of the cross section of the plate (40) are 0,5 cm to 3 cm, more preferably 1 cm to 2.5 cm.

Each of the dimensions of the cross section of the plate (40) may be 1 cm, 1.1 cm, 1.2 cm, 1.3 cm, 1.4 cm, 1.5 cm, 1.6 cm, 1.7 cm, 1.8 cm, 1.9 cm, 2 cm, 2.1 cm, 2.2 cm, 2.3 cm, 2.4 cm or 2.5 cm.

Preferably, the height of the plate (40) is 10 to 25 mm, more preferably 15 to 20 mm.

The height of the plate (40) may be 15 mm, 16 mm, 17 mm, 18 mm, 19 mm or 20 mm.

The cover (C1) is preferably fluid non-permeable. Preferably the fluid is water.

Preferably, fluid non-permeable means that at least 90 wt.-%, more preferably at least 95 wt.-%, most preferably at least 99 wt.-%, particularly preferred 100 wt.-% of the fluid which is part of the matrix (M1) does not pass through the cover (C1).

Preferably, at least one of the at least one cover (C1) and/or several covers (C1) or each cover (C1) comprise the shape of the corresponding mesh(es) (20), which preferably is/are covered by the cover(s) (C1).
Preferably, at least one of the at least one cover (C1) and/or several covers (C1) or each cover (C1) comprise(s) a height and/or a cross section.

Preferably, the dimensions of at least one of the at least one cover (C1) are such that they may ensure tight connection with mesh insert (20).

Preferably, the height of at least one of the at least one cover (C1) and/or several covers (C1) or each cover (C1) is the same as or different from the corresponding mesh (20), which is covered by the cover (C1).

Preferably at least one of the dimensions of cross section of each/or several/or at least one of the at least one cover (C1) is 0.05 mm to 2,5 mm, more preferably 0.1 mm to 2 mm larger than the corresponding dimension of cross section of the mesh (20).

At least one of the dimensions of cross section of each/or several/or at least one of the at least one cover (C1) may be 0.1 mm, 0.2 mm, 0.3 mm, 0.4 mm, 0.5 mm, 0.6 mm, 0.7 mm, 0.8 mm, 0.9 mm, 1 mm, 1.1 mm, 1.2 mm, 1.3 mm, 1.4 mm, 1.5 mm, 1.6 mm, 1.7 mm, 1.8 mm, 1.9 mm or 2 mm larger than the corresponding dimension of cross section of the mesh (20).

For example in a rectangular mesh with a rectangular cover (C1), the length and/or the width of cross section of at least one cover (C1) may be 0.1 mm, 0.2 mm, 0.3 mm, 0.4 mm, 0.5 mm, 0.6 mm, 0.7 mm, 0.8 mm, 0.9 mm, 1 mm, 1.1 mm, 1.2 mm, 1.3 mm, 1.4 mm, 1.5 mm, 1.6 mm, 1.7 mm, 1.8 mm, 1.9 mm or 2 mm larger than the length or the width of the mesh (20).

Preferably, the height of at least one of the at least one cover (C1) may be at least the same as the height of the at least one of the at least one mesh (20).

Preferably, the cover (C1) may comprise a holder (10).

Preferably, material of the holder may be polystyrene, polycarbonate or polypropylene.

Preferably, the holder (10) may be square column or round column.

Preferably, the holder (10) may have a width and a height.

Preferably, the widths and/or the height of the holder (10) may be 1,5 cm to 2.5 cm, more preferably 1.7 cm to 2 cm.

Preferably, the widths and/or the height of the holder (10) may be 1.5, 1.6, 1.7, 1.8, 1.9, 2.0, 2.1, 2.2, 2.3, 2.4 or 2.5 cm.

Preferably, the width and/or the height of the holder (10) may be selected so that to be easy grip by hand.

Preferably, the cover (C1) may be removable.

The holder (10) may be used to take the cover (C1) in order to insert the cover (C1) around the mesh (20) and/or remove the cover (C1) from the mesh (20).

Preferably, the mesh (20) may comprise a rim (30) with a contact surface.

Preferably, material of the rim (30) may be polystyrene, polycarbonate and/or polypropylene.

Preferably, material of the holder (10) may be the same or different from material of the rim (30).

Preferably, the rim (30) may be circular, triangular, rectangular, rhombic, oval, square or parallelogram, symmetric or asymmetric.

Preferably, the rim (30) comprises a width.

Preferably, the width of the rim (30) may be 1 to 4 mm, more preferably, 2 to 3 mm.

Preferably, the width of the rim (30) may be 1, 1.5, 2, 2.5, 3, 3.5 or 4 mm.

Preferably, the contact surface of the rim (30) may be coated with an adhesive.

The adhesive may be at least partially covered by a protection cover (PC1).

The protection cover (PC1) on the rim allows user to "stick" the mesh and the rim on selected part of the plate, according to desires and needs of the user.

Preferably, the widths of the rim (30) may be selected so that to ensure sufficient contact area for adhesion with "host" plate (40).

Preferably, the adhesive may be a silicone adhesive and/or a pressure sensitive adhesive; more preferably the adhesive may be protected by PET (polyethylene terephthalate) liner.

Especial pressure sensitive adhesive may be tacky at room temperature and adhere firmly to surfaces after pressure is applied.

Preferably, pressure sensitive adhesives may be commercially available MA-69 acrylic hybrid medical grade adhesive or SR-26 silicone adhesive. Both adhesives have passed cytotoxicity tests conducted by an independent laboratory.

Preferably, at least one of the at least one mesh (20) may comprise organic materials, more preferably Nylon.

Preferably, the device may comprise matrix (M1) in at least one of the at least one mesh (20).

The matrix (M1) may be solidifyable. The matrix (M1) preferably comprises a synthetic matrix for 3D cell culture and/or a mixture of cell culture medium and gelatinous extract of extracellular matrix proteins isolated from Engelbreth-Holm-Swarm (EHS) mouse sarcomas; wherein preferably synthetic matrix for 3 dimensional cell culture is selected from Dulbecco's Modified Eagle Medium (DMEM), Minimum Essential Medium (MEM), RPMI-1640, Iscove's Modified Dulbecco's Medium (IMDM), Dulbecco's Modified Eagle Medium/Nutrient Mixture F-12 (DMEM/F-12) and/or RPMI 1640.

Preferably, the matrix (M1) in each of the mesh (20) may be the same or different.

Preferably, each mesh (20) may be different in size and/or shape.

Preferably, each mesh (20) may be different in size and/or each mesh (20) may be different in shape.

Preferably, all or several meshes (20) may have the same shape and/or the same size.

Preferably, the at least one of the at least one mesh (20) may be a ring, a cylinder, a non-cylinder, wherein preferably at least one cross section of at least one of the at least one mesh (20) is a circle, triangle, rectangle, rhomb, oval, square or parallelogram, symmetric or asymmetric.

Preferably, the number of the mesh (20) and/or the plate (40) may be more than one; more preferably the number of the meshes (20) may the same as the plates (40) or may different from the plates (40).

Preferably, the number the mesh (20) and/or the plate (40) may be at most 30.

Preferably, the matrix (M1) may further comprise cells to be cultured.

It should be understood that the number of mesh (20) in at least one/or more plate (40) or at each plate (40) may be 2, 3 ,4, 5, 6, 7, 8, 9, 10, 11, 12, 13, 14, 15, 16, 17, 18, 19, 20, 21, 22, 23, 24, 25, 26, 27, 28, 29, 30.

According to a further embodiment of the device, at least one of the at least one cover (C1) may be a foil or a liner.

Preferably, the foil or the liner may comprise a metal, preferably Aluminium.

Preferably, the foil or the liner may comprise a plastic, preferably polyethylene terephthalate (PET), and/or polyethylene (PE), and/or Polyvinyl chloride (PVC).

Preferably, the foil or the liner may comprise a metal and a plastic, more preferably polyethylene terephthalate (PET), and/or polyethylene (PE), and/or Polyvinyl chloride (PVC), and/or Aluminium.

Preferably, the foil or the liner may comprise at least one or more than one holder (10).

Preferably, the foil or the liner may be removable.

According to a further embodiment of the device, at least one of the at least one mesh (20) may comprise a rim (30) with a contact surface.

According to a further embodiment of the device, the contact surface of the rim (30) may be coated with an adhesive; preferably the adhesive may be a silicone adhesive and/or a pressure sensitive adhesive.

According to a further embodiment of the device, the adhesive may be at least partially covered by a protection cover (PC1); more preferably the protection cover (PC1) may be a PET (polyethylene terephthalate) liner or foil.

Further, the invention is directed to a method for three dimensional (3D) culture of cells in a matrix (M1) comprising the steps:
(a) installing at least one mesh in at least one plate;
(b) inserting at least one cover (C1) around the at least one mesh (20);
(c) adding at least one matrix (M1) inside at least one of the at least one mesh (20) and/or at least one of the at least one mesh (20) may comprise at least one matrix (M1);
(d) removing at least one of the at least one cover (C1) after solidifying of the at least one matrix (M1);
(e) adding at least one matrix (M2) into at least one of the at least one plate (40) outside the area enclosed by at least one of the at least one mesh (20).

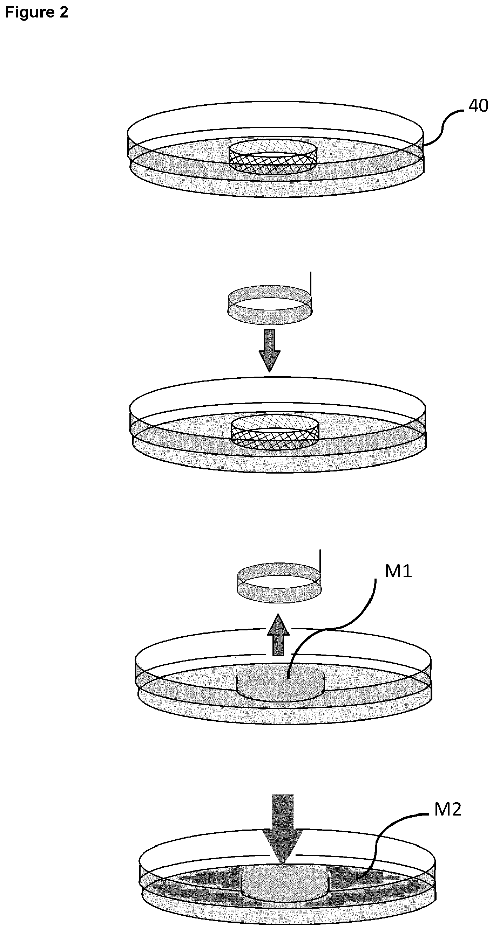

Fig. 2 shows one embodiment of the inventive method.

M2 matrix may be typically a cell culture medium and may be in liquid form that may provide nutrients and growth factors essential for cells, growing in form of spheres inside the mesh (20) inserts. Changing the medium (by vacuum aspiration) may not disturb the cells that are located inside the mesh (20).

It should be understood that several matrices (M2) may be added in at least one/or at each one of plate (40) outside the area enclosed by at least one of the at least one mesh (20).

The mesh from hardened mesh material with relative large dimension of the pores in which matrices (M1) and (M2) are in contact through from the lateral direction of the mesh allows faster exchange of nutrients and gases between cell culture medium and the gelatinous extract of extracellular matrix proteins isolated from Engelbreth-Holm-Swarm (EHS) mouse sarcomas (for example Matrigel).

The lateral contact direction eases contact of matrices M1 and M2, in comparison with the conventional devices, in which the mesh is on bottom of a container well. Placement of the mesh on bottom of the well results in disturbance of the contact of the cell culturing matrices, due to the gas bubbles, produced while cell culturing. The rigidity and resistance to mechanical stress or mechanical strength factors and gas exchange property of the gelatinous extract of extracellular matrix proteins isolated from Engelbreth-Holm-Swarm (EHS) mouse sarcomas may depend on concentration of the gelatinous extract of extracellular matrix proteins isolated from Engelbreth-Holm-Swarm (EHS) mouse sarcomas. Thus, the device, design and method disclosed herein specifically eases usage of gelatinous extract of extracellular matrix proteins isolated from Engelbreth-Holm-Swarm (EHS) mouse sarcomas for 3D cell culture, which is one of the most used approaches in stem cell and cancer research.

The mesh is an organic part of the plate, which is "imprinted on the plate" and not on bottom of a well. Matrices M1 and M2 are in contact through the mesh in lateral direction. Thus, it is enabled to take images of cells/spheroids without disturbance of infrared, visible, ultraviolet or fluorescent light by a mesh material, placed in the path of the light source. Furthermore, organic material of the mesh strongly reduces the reflection of the incoming beam, which could happen by using for example a metallic mesh.

The mesh frame disclosed by method and device of the invention is an organic part of the plate. Therefore, the risk of infection and contamination of the cell culture media, which is closely linked with manipulation and placing of external devices, is eliminated by using the device as well as the method disclosed herein.

The inventive device as well as the inventive method allows user to change the cell culture medium without disturbing the gelatinous extract of extracellular matrix proteins isolated from Engelbreth-Holm-Swarm (EHS) mouse sarcomas with embedded cells/spheres. Furthermore, the mesh allows penetration of oxygen and nutrients into cells embedded in the gelatinous medium. Therefore, the device as well as method disclosed herein minimizes loss of valuable cultured cells, which occurs while changing of the culture media via conventional methods like for example using a pipette, due to semi-solid nature of the gelatinous extract of extracellular matrix proteins isolated from Engelbreth-Holm-Swarm (EHS) mouse sarcomas.

According to a further embodiment of the device and the method, preferably the device comprises matrix (M1) in the at least one of the at least one mesh (20).

According to a further embodiment of the device and the method, preferably the matrix (M1) comprises cells to be cultured and cell culture medium.

According to a further embodiment of the device and the method, preferably the cell culture medium comprises:
(a) matrix (M2) or
(b) matrix (M3) or
(c) matrix (M2) and gelatinous extract of extracellular matrix proteins isolated from Engelbreth-Holm-Swarm (EHS) mouse sarcomas.

According to a further embodiment of the device and the method, preferably the matrix (M1) is selected to be solidifyable, preferably if the diameter of the cells to be cultured is smaller than the pores dimension of the mesh (20).

According to a further embodiment of the device and the method, preferably the matrix (M1) is solidifyable if (M1) comprises
(a) matrix (M3), or
(b) matrix (M2) and gelatinous extract of extracellular matrix proteins isolated from Engelbreth-Holm-Swarm (EHS) mouse sarcomas.

According to a further embodiment of the device and the method, preferably matrix (M2) is preferably liquid.

According to a further embodiment of the device and the method, preferably matrix (M2) comprises Dulbecco's Modified Eagle Medium (DMEM), Minimum Essential Medium (MEM), RPMI-1640, Iscove's Modified Dulbecco's Medium (IMDM), Dulbecco's Modified Eagle Medium/Nutrient Mixture F-12 (DMEM/F-12) and/or RPMI 1640.

According to a further embodiment of the device and the method, preferably matrix (M3) comprises hydrogels, preferably poly (ethylene glycol) (PEG), poly(vinyl alcohol) and/or poly(2-hydroxy ethyl methacrylate) hydrogels.

According to a further embodiment of the device and the method, preferably at least one of the at least one mesh (20) may comprise organic material, more preferably Nylon.

According to a further embodiment of the device and the method, preferably matrix (M1) may be a solidifyable material; more preferably, matrix (M1) may be a synthetic matrix for 3 dimensional cell culture and/or a mixture of cell culture medium and gelatinous extract of extracellular matrix proteins isolated from Engelbreth-Holm-Swarm (EHS) mouse sarcomas.

According to a further embodiment of the device and the method, preferably at least one of the at least one matrix (M2) may comprise a synthetic matrix for 3D cell culture and/or a mixture of cell culture medium.

According to a further embodiment of the device and the method, preferably at least one of the at least one matrix (M2) may comprise a liquid.

According to a further embodiment of the device and the method, preferably at least one of the at least one matrix (M2) may comprise Dulbecco's Modified Eagle Medium (DMEM), Minimum Essential Medium (MEM), RPMI-1640, Iscove's Modified Dulbecco's Medium (IMDM), Dulbecco's Modified Eagle Medium/Nutrient Mixture F-12 (DMEM/F-12) and/or RPMI 1640.

According to a further embodiment of the method, installing at least one of the at least one mesh (20) in at least one of the at least one plate (40) may comprise detaching the protection cover (PC1) from the rim (30).

According to a further embodiment of the method, installing at least one of the at least one mesh (20) in at least one of the at least one plate (40) may comprise attaching and/or pressing the rim (30) to at least one of the at least one plate (40).

According to a further embodiment of the device and the method, at least one of the at least one cover (C1) may comprise a holder (10).

According to a further embodiment of the method, inserting and/or removing at least one of the at least one cover (C1) may be done by using the holder (10).

### REFERENCES

(1) https://www.corning.com/worldwide/en/products/life-sciences/products/surfaces/matrigel-matrix.html
(2) https://www.adhesivesresearch.com/wp-content/uploads/2013/11/90106-Data-Sheet.pdf
(3) https://www.adhesivesresearch.com/wp-content/uploads/2019/04/90880-Data-Sheet.pdf

## Claims

1. A device for three dimensional (3D) culture of cells in a matrix (M1) comprises:
(a) at least one mesh (20) inserted in at least one plate (40)
(b) at least one cover (C1) around the at least one mesh (20) ; which preferably is fluid non-permeable.

2. A method for three dimensional (3D) culture of cells in a matrix (M1) comprising the steps:
(a) installing at least one mesh (20) in at least one plate (40);
(b) inserting at least one cover (C1) around the at least one mesh (20);
(c) adding at least one matrix (M1) inside at least one of the at least one mesh (20); and/or at least one of the at least one mesh (20) comprises at least one matrix (M1);
(d) removing at least one of the at least one cover (C1) after solidifying of the at least one matrix (M1);
(e) adding at least one matrix (M2) into at least one of the at least one plate (40) outside the area enclosed by at least one of the at least one mesh (20).

3. The device of claim 1, wherein at least one of the at least one mesh (20) comprises a cross section, which preferably is in contact with at least one of the at least one plate (40), and a height, wherein preferably
(a) each of the dimensions of the cross section is 0.5 cm, 1 cm, 1.5 cm, 2 cm; and/or
(b) the height of at least one of the at least one mesh (20) is less than the height of at least one of the at least one plate (40), more preferably the height of at least one of the at least one mesh (20) is 10 mm or 15 mm; and/or
(c) each of the dimensions of the mesh (20) pores is 10 µm, 20 µm, 30 µm, 40 µm, 50 µm, 60 µm, 70 µm, 80 µm, 90 µm, 100 µm; and/or
(d) at least one of the at least one plate (40) is made of clear polystyrene; and/or
(e) at least one of the at least one plate (40) has a cross section and a height; and/or
(f) each of the dimensions of the cross section of at least one of the at least one plate (40) is 1 cm, 1.5 cm, 2 cm, 2.5 cm; and/or
(g) the height of at least one of the at least one plate (40) is 15 mm or 20 mm; and/or
(h) at least one of the at least one cover (C1) and/or several covers (C1) or each cover (C1) comprise(s) a height and/or a cross section; and/or
(i) the dimensions of at least one of the at least one cover (C1) are such that they ensure tight connection with mesh insert (20), more preferably at least one of the dimensions of cross section of each/or several/or at least one of the at least one cover (C1) is 0.1 mm, 0.2 mm, 0.3 mm, 0.4 mm, 0.5 mm, 0.6 mm, 0.7 mm, 0.8 mm, 0.9 mm, 1 mm, 1.1 mm, 1.2 mm, 1.3 mm, 1.4 mm, 1.5 mm, 1.6 mm, 1.7 mm, 1.8 mm, 1.9 mm or 2 mm larger than the corresponding dimension of cross section of the mesh (20); and/or the height of at least one of the at least one cover (C1) is the same as, at least the same as or different from the height of the at least one of the at least one mesh (20).

4. The device and the method of any of previous claims, wherein
(I) the device comprises matrix (M1) in the at least one of the at least one mesh (20); and/or
(II) the matrix (M1) comprises cells to be cultured and cell culture medium and/or
(III) the cell culture medium comprises
(a) matrix (M2) or
(b) matrix (M3) or
(c) matrix (M2) and gelatinous extract of extracellular matrix proteins isolated from Engelbreth-Holm-Swarm (EHS) mouse sarcomas and/or
(IV) the matrix (M1) is selected to be solidifyable , preferably if the diameter of the cells to be cultured is smaller than the pores dimension of the mesh (20) and/or
(V) the matrix (M1) is solidifyable if (M1) comprises
(a) matrix (M3), or
(b) matrix (M2) and gelatinous extract of extracellular matrix proteins isolated from Engelbreth-Holm-Swarm (EHS) mouse sarcomas; and/or
(VI) matrix (M2) is preferably liquid; and/or
(VII) matrix (M2) comprises Dulbecco's Modified Eagle Medium (DMEM), Minimum Essential Medium (MEM), RPMI-1640, Iscove's Modified Dulbecco's Medium (IMDM), Dulbecco's Modified Eagle Medium/Nutrient Mixture F-12 (DMEM/F-12) and/or RPMI 1640; and/or
(VIII) matrix (M3) comprises hydrogels, preferably poly (ethylene glycol) (PEG), poly(vinyl alcohol) and/or poly(2-hydroxy ethyl methacrylate) hydrogels.

5. The device and the method of any of previous claims, wherein
(a) at least one of the at least one mesh (20) comprises organic materials, preferably Nylon; and/or
(b) the matrix (M1) in each of the mesh (20) is the same or different; and/or
(c) each mesh (20) is different in size and/or shape; and/or each mesh (20) is different in size and/or each mesh (20) is different in shape; and/or all or several meshes (20) have the same shape and/or the same size; and/or
(d) the at least one of the at least one mesh (20) is a ring, a cylinder, a non-cylinder, wherein preferably at least one cross section of at least one of the at least one mesh (20) is a circle, triangle, rectangle, rhomb, oval, square, parallelogram, symmetric or asymmetric; and/or
(e) the number of the mesh (20) and/or the plate (40) is more than one; wherein preferably
i) the number of the meshes (20) is the same as the plates (40); and/or is different from the plates (40); and/or
ii) the number the mesh (20) and/or the plate (40) is at most 30; and/or
iii) the number of the meshes (20) in at least one plate (40) and/or the number of the plates (40) is 2, 3 ,4, 5, 6, 7, 8, 9, 10, 11, 12, 13, 14, 15, 16, 17, 18, 19, 20, 21, 22, 23, 24, 25, 26, 27, 28, 29, or 30.

6. The device and the method of any of previous claims, wherein at least one of the at least one cover (C1)
(a) is a foil and/or a liner, wherein optionally the foil and/or the liner comprises a metal, preferably Aluminium; and/or a plastic, preferably polyethylene terephthalate (PET), and/or polyethylene (PE), and/or Polyvinyl chloride (PVC); and/or
(b) comprises a holder (10); and/or
(c) is removable.

7. The device and the method of any of previous claims, wherein at least one of the at least one mesh (20) comprises a rim (30) with a contact surface and/or a width, wherein preferably
(a) the rim (30) may be circular, triangular, rectangular, rhombic, oval, square or parallelogram, symmetric or asymmetric; and/or
(b) the width of the rim (30) is 1 to 4 mm, more preferably, 2 to 3 mm; and/or
(c) the width of the rim (30) is 1, 1.5, 2, 2.5, 3, 3.5 or 4 mm.

8. The device and the method of claim 7, wherein the contact surface of the rim (30) is coated with an adhesive; preferably the adhesive is a silicone adhesive and/or a pressure sensitive adhesive.

9. The device and the method of claim 8, wherein the adhesive is at least partially covered by a protection cover (PC1); wherein preferably the protection cover (PC1) is a PET (polyethylene terephthalate) liner or foil.

10. The method of claim 9, wherein installing at least one of the at least one mesh (20) in at least one of the at least one plate (40) comprises:
(a) detaching the protection cover (PC1) from the rim (30); and/or
(b) attaching and/or pressing the rim (30) to at least one of the at least one plate (40).

11. The device and the method of any of previous claims, wherein at least one of the at least one cover (C1) comprises a holder (10); wherein preferably;
(a) material of the holder (10) is the same or different from material of the rim (30); and/or
(b) the holder (10) is square column or round column; and/or
(c) the holder (10) has a width and a height; wherein more preferably
(i) the width and/or the height of the holder (10) are/is 1,5 cm to 2.5 cm, most preferably 1.7 cm to 2 cm; and/or
(ii) the widths and/or the height of the holder (10) are/is 1.5, 1.6, 1.7, 1.8, 1.9, 2.0, 2.1, 2.2, 2.3, 2.4 or 2.5 cm.

12. The method of claim 11, wherein inserting and/or removing at least one of the at least one cover (C1) is done by using the holder (10).
